# NEW EUROPEAN PATENT SPECIFICATION

(11) **EP 1 813 239 B2**
(45) Date of publication and mention of the opposition decision: **30.08.2017**
(45) Mention of the grant of the patent: 07.05.2014
(21) Application number: 04788416.8
(22) Date of filing: 30.09.2004
(51) Int. Cl.: A61F 13/475, A61F 13/536, A61F 13/532, A61F 13/534

(54) **ABSORPTIVE ARTICLE**
SAUGFÄHIGER ARTIKEL
ARTICLE ABSORBEUR

(43) Date of publication of application: 01.08.2007
(73) Proprietor: DAIO PAPER CORPORATION, Shikokuchuo-shi, Ehime 799-0492 (JP)
(72) Inventor: GOTO, Iwao, DAIO SANITARY CO., LTD., Fuji-shi, Shizuoka 4190201 (JP)
(74) Representative: Henkel, Breuer & Partner
(86) International application number: PCT/JP2004/014388
(87) International publication number: WO 2006/038264

(56) References cited:
- EP-A1- 0 067 916
- WO-A1-00/71790
- CN-Y- 2 527 254
- CN-Y- 2 548 609
- JP-A- 10 137 291
- JP-A- 57 035 001
- JP-A- 2004 049 696
- JP-A- 2004 298 330
- JP-U- 61 204 726
- JP-Y2- 59 025 369
- US-A- 4 055 180
- US-A- 5 514 120
- US-A- 5 938 650
- US-A- 5 994 614
- US-A1- 2002 102 392
- US-B1- 6 221 460

## Description

### Technical Field

The present invention relates to an absorptive article to be mainly used as a sanitary napkin, a vaginal discharge sheet, an incontinence pad, a medical pad or a toiletry and, more particularly, to an absorptive article for providing softness to a skin and for preventing the cellulose wadding of the absorptive article from being displaced or cracked even in the case where an absorbing body is thin.

### Background Art

In the prior art, there is known an absorptive article such as a panty liner, a sanitary napkin or an incontinence pad, in which an absorbing body made of a cellulose wadding (containing a highly water-absorptive resin mixed therein) of pulverized pulp or the like is interposed between a liquid impermeable rear surface sheet such as a polyethylene sheet or a polyethylene sheet laminate nonwoven fabric and a liquid permeable front surface sheet such as a nonwoven fabric or a liquid permeable plastic sheet.

In recent years, the case of a relatively bulky absorptive article raises a problem that the article is inconvenient to carry or poor in accommodation. From the requirement for efficient physical distribution and for conservation of natural resources, moreover, it is the tendency to prefer a thin absorptive article, in which the absorbing body in the bodily liquid discharging portion has a thickness of 8 mm or less, preferably 5 mm or less. At the time of thinning the absorptive article, it is impossible to reduce the water absorption cannot be lowered even the article is made thin and compact. It is, therefore, necessarily frequent to increase the content of a highly water-absorptive resin and to use a polymer sheet. At the same time, the absorptive article is thinned by pressing it severely (as referred to in JP-2003-38552A and in JP-2003-38574A.

WO 00/71790 A1 discloses a fibrous absorbent article in the form of a web that has upper and lower layers of with matrix fibers of cellulosic or synthetic fibers containing a thermoplastic bonding material. Sandwiched between the upper and lower layers is a functional particle area applied in substantially square zones or parallel lanes and disposed so that it is in contact with the upper and lower layers. Seals are formed by binding the upper and lower layers in the particle-free zones in that the structure is compacted by pressure and heat.

US 5938650 A discloses an absorbent core for absorbing body liquids that includes two thin outer layers with at least one layer being porous and receptive to body liquids. A quantity of superabsorbent polymer particles is provided between the outer layers and is loosely contained in individual unbonded zones of flat closed pockets defined by a plurality of intersecting and uninterrupted heat bond lines between the layers forming a regular grid. The bond lines are formed by applying heat and pressure through a calendar and define wicking lines between the pockets.

### Disclosure of the Invention

However, the absorptive article (or the pulverized pulp) thinned by pressing it has its density raised so extremely the wearer feels stiffness of the absorptive article. Moreover, the cellulose wadding restores an increased thickness after produced, and the absorbing body is thin and contains a highly water-absorptive resin in a large quantity, so that it is troubled by an easy displacement or cracking of the cellulose wadding. Still moreover, the absorbing body is highly densified so that the bodily liquid is seriously diffused by the capillarity. As a result, there arises a problem that a feeling of anxiety is given to the transverse leakage of the absorbing body in the short side direction (or the transverse direction).

It is, therefore, a major object of the invention is to provide an absorptive article, which can provide softness to a skin when the absorptive article is put thereon, thereby to give a satisfactory wear feeling, and to prevent the cellulose wadding of the absorptive article from being displaced or cracked even in the thin absorptive article liable to become hard while suppressing the dispersion of a bodily liquid in the widthwise direction thereof.

### Means for Solving the Problems

In order to solve the problems, according to the invention, there is provided an absorptive article comprising the features of claim 1.

In the invention, in order to soften the feeling of the relatively thin but hard, the thermally adhesive fabric layers are disposed individually on the upper and lower surface sides of the absorbing bodies, and the embosses are added to join the thermally adhesive fabric layer on the upper surface side of the absorbing bodies and the thermally adhesive fabric layer on the lower surface side to each other. As a result, the presence of the thermally adhesive fabric layers gives the softness and the cushioning properties to the skin touching side and the side surface. As a result, the wear feeling is improved, and the positional displacement of the absorbing body is prevented by the join between the thermally adhesive fabric layers thereby to prevent the displacement and the cracking of the cellulose wadding. At the same time, it is also possible to prevent the pulp from being restored to increase the thickness. As an accompanying advantage, moreover, the shielding properties can be improved by the thermally adhesive fabric layers arranged on the skin untouching side, thereby to make the menstrual blood or the like neutral.

In the invention, the embosses are the plural lines of linear embosses added to the absorptive article in the direction along the approximately longitudinal direction of the absorptive article. By these linear embosses, the bodily liquid is prevented from being diffused in the widthwise direction, and the linear embosses act as hinge points so that the absorbing bodies become deformable. As a result, the linear embosses touches on the roots of the legs become weak so that the uncomfortable feeling is reduced to improve the wear feeling. Here, the linear embosses may be continuous lines or intermittent lines.

In a preferred embodiment said plural lines of linear embosses have a spacing of 1 to 30 mm, and wherein the embosses have a bottom groove width of 0.05 to 5 mm. The linear embosses are desirably added within the numerical range so as to give the softness to the skin, when the absorptive article is worn, to improve the wear feeling, and to prevent the displacement and cracking of the cellulose wadding of the absorptive article.

In the invention, said plural lines of linear embosses reach wholly or mostly the peripheral edge of the absorbing body at the front and rear end portions of the absorbing body, while drawing curves in the directions to leave the longitudinal center line gradually. It is desired that the linear embosses are aligned with the extending direction of the shorts. Our tests have revealed that the linear embosses are desired to have draw such patterns at the front and rear end portions of the absorbing body that they reach the peripheral edge of the absorbing body while drawing curves in the directions to leave the longitudinal center line gradually outward.

In a preferred embodiment a nonwoven fabric is disposed as said thermally adhesive fabric layers, and wherein said absorbing body is peripherally wrapped by a single sheet.

In a preferred embodiment the pulp of the absorbing body is highly densified at said joined portions.

### Advantage of the Invention

According to the invention, as has been described in detail hereinbefore, it is possible to provide an absorptive article, which can provide softness to a skin when the absorptive article is put on, thereby to give a satisfactory wear feeling, and to prevent the cellulose wadding of the absorptive article from being displaced or cracked even in the thin absorptive article liable to become hard while suppressing the dispersion of a bodily liquid in the widthwise direction thereof.

### Best Mode for Carrying Out the Invention

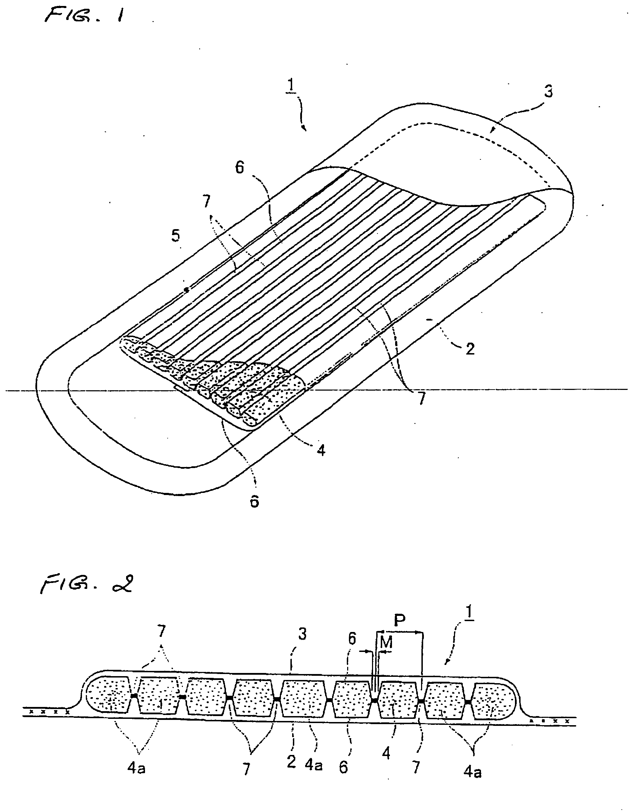

A mode of embodiment of the invention is described in detail in the following with reference to the accompanying drawings. Fig. 1 is a partially broken perspective view of a thin sanitary napkin 1 according to the invention.

This thin sanitary napkin 1 is made available mainly for application to a panty liner, a sanitary napkin, a vaginal discharge sheet or an incontinence pad. As shown in Fig. 1, for example, absorbing bodies 4 are interposed between a liquid impermeable rear surface sheet 2 and a liquid permeable front surface sheet 3, and thermally adhesive fabric layers 6 and 6 are individually disposed on the upper and lower surface sides and embossed to join the thermally adhesive fabric layer 6 disposed on the upper surface side of the absorbing bodies and the thermally adhesive fabric layer 6 disposed on the lower surface side of the absorbing bodies. Around the absorbing bodies 4, the liquid impermeable rear surface sheet 2 and the liquid permeable front surface sheet 3 are joined by adhesive means such as a hot melt adhesive.

The liquid impermeable rear surface sheet 2 is made of a sheet material having at least water-repellent properties such as polyethylene or polypropylene, and may also be made of a nonwoven fabric sheet (in this case, the liquid impermeable rear surface sheet is composed of a waterproof film and a nonwoven fabric) or the like while keeping the liquid impermeable properties substantially by interposing a waterproof film. In recent years, from the viewpoint of preventing stuffiness, it is a tendency to use a moisture permeable material. These water-repellent/moisture-permeable sheet materials are preferably made of porous sheets, which are obtained by melting and blending an inorganic filler in an olefin resin such as polyethylene or polypropylene to form a sheet and then by orienting the sheet uniaxially or biaxially.

The liquid permeable front surface sheet 3 is preferably made of a non-porous or porous nonwoven fabric or a porous plastic sheet. The material fibers composing the nonwoven fabric can be synthetic fibers of an olefin family of polyethylene or polypropylene, a polyester family or a polyamide family, regenerated fibers such as rayon or cupro-ammonium rayon, or natural fibers such as cotton. Other material fibers can also be composite fibers such as core-sheath fibers made of cores of fibers having a high melting point and sheaths of fibers having a low melting point, side-by-side fibers, or split type fibers.

The nonwoven fabric can also be worked by a suitable method such as a spun-lace method, a spun-bond method, a thermal-bond method, a melt-brown method, a needle-punch method or an air-laid method. Of these working methods, the spun-lace method is excellent for a product having rich draping properties, and the thermal-bond method is excellent for a bulky and soft product.

The absorbing bodies 4 interposed between the liquid impermeable rear surface sheet 2 and the liquid permeable front surface sheet 3 are prepared, for example, by mixing a high water-absorptive resin into pulp, or by mixing not only chemical fibers but also a high water-absorptive resin into pulp. The absorbing bodies 4 can also be enclosed by (not-shown) crepe paper so as to hold the shape, to diffuse menstrual blood promptly, and to prevent the backward flow of the menstrual blood once absorbed.

Especially in the case of the thin sanitary napkin 1 of the invention, the absorbing bodies 4 are thinned by pressing them. The target thickness of the absorbing bodies 4 in the bodily liquid discharging portion (including the case, in which a high center portion is formed) is set to 8 mm or less, preferably 5 mm or less, or more preferably 3 mm or less. The top plan shape of the absorbing bodies 4 may be an oval shape, as shown, or a fit-cut shape (or a gourd shape) so as to provide softness to a crotch.

The pulp is made of chemical pulp obtained from wood, such as molten pulp, mechanical pulp, paper making pulp, dissolved pulp or synthetic pulp, cellulose fibers such as chemical fibers, or artificial cellulose fibers such as rayon or acetate. Conifer pulp having longer fibers than hose of hard wood pulp is preferably used in the aspects of functions and price.

The high water-absorptive resin can be exemplified by a saponifier of crosslinked polyacrylate, self-crosslinked polyacrylate or copolymer of acrylic-ester-vinylacetate, crosslinked isobutylene/maleic-anhydride, crosslinked polysulfonate, or partially crosslinked water-growing polymer such as polyethylene oxide or polyacryl amide. Of these, the acrylic acid or acrylate family is preferred because it is excellent in its water absorptivity and water absorbing rate. The highly water-absorptive resin having the water absorbing performance can be adjusted in its production process on the water absorptivity and the water absorbing rate by adjusting the crosslinked density and the crosslinked density gradient. The content of the highly water-absorptive resin is desired to be 10 to 60 %. A sufficient absorptivity cannot be given, in case the content of the highly water-absorptive resin is less than 10 %. In case the content is more than 60 %, the interlace between the pulp fibers disappears so that the sheet intensity drops to invite breakage and cracking easily.

The thermally adhesive fabric layers 6 and 6, as individually disposed between the liquid permeable front surface sheet 3 and the absorbing bodies 4 and between the liquid impermeable rear surface sheet 2 and the absorbing bodies 4, can be preferably made of the nonwoven fabric.

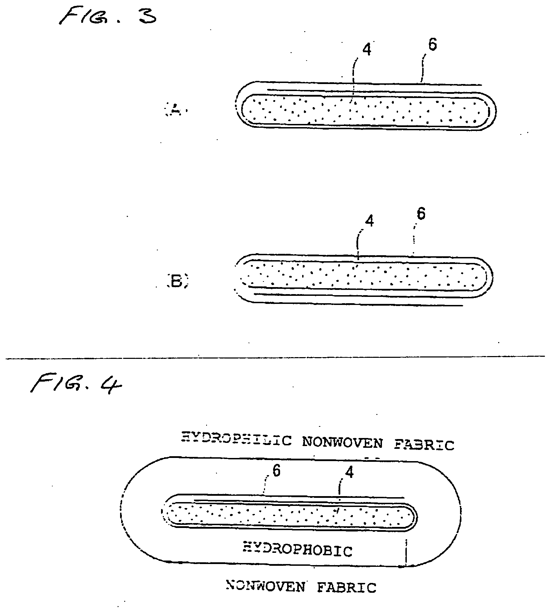

In another mode of disposing the nonwoven fabrics 6 and 6, the two separate nonwoven fabrics 6 and 6 may also be individually disposed between the liquid permeable front surface sheet 3 and the absorbing body 4 and between the liquid impermeable rear surface sheet 2 and the absorbing body 4. It is, however, desired that the two nonwoven fabrics 6 and 6 are arranged to enclose the periphery of the absorbing bodies 4, as shown in Fig. 3. Fig. 3(A) is a transverse section of the case, in which the nonwoven fabric 6 wraps the absorbing body 4 with its end portions being overlapped on the upper surface side, and Fig. 3(B) is a transverse section of the case, in which the nonwoven fabric 6 wraps the absorbing body 4 with its end portions being overlapped on the lower surface side. In the case of the upper wrapping case, the two nonwoven fabrics are overlapped on the skin surface side so that they can provide higher softness. In the case of the lower wrapping case, on the other hand, the shielding properties on the skin untouching side can be better improved.

The nonwoven fabric 6 is given the hydrophilic properties either by using the regenerated fibers of rayon or cupro-ammonium rayon, or natural fibers such as cotton thereby to use the material having the hydrophilic properties by itself, or by using the fibers which are prepared by treating synthetic resins of the olefin family such as polyethylene or polypropylene, the polyester family or the polyamide family on their surfaces with a hydrophilic agent thereby to give the hydrophilic properties. Moreover, the nonwoven fabric 6, as disposed between the liquid impermeable rear surface sheet 2 and the absorbing body 4, may be either hydrophilic or hydrophobic, but is preferred to be hydrophobic. In case the absorbing body 4 is wrapped by a single sheet, as described above, it is desired that the sheet for the skin surface side is treated to have the hydrophilic properties whereas the sheet for the skin untouching side is treated to have the hydrophobic properties. In other words, the area on the skin untouching side is treated to have the water repellent properties, in case the material itself has the hydrophilic properties, and the area on the skin surface side is treated to have the hydrophilic properties, in case the material itself has the water-repellent properties, thereby to give the skin side area and the skin untouching side area the different properties.

It is desired to use such a nonwoven fabric, although made different physical properties by the preparing method, as gives, when worn, the skin touching side and/or the skin untouching side the softness (or the soft feeling) thereby to make the wear feeling good and to enhance the shielding properties on the skin untouching side. The nonwoven fabric satisfying that condition may be exemplified by an air-through nonwoven fabric. The air-through nonwoven fabric is enabled, by its bulkiness, to improve the cushioning properties and, by its retention of the distance from the outer surface side, to improve the shielding properties. In order to provide the softness to the skin, moreover, it is desired that the nonwoven fabric has a basis weight of 10 g/m² or higher, preferably 15 g/m² or higher, or more preferably 20 g/m² or higher. Here, the nonwoven fabric 6 and the absorbing body 4 are desirably joined by a hot-melt adhesive, but may also be given the later-described embosses 7 while being not joined.

In the present absorbing body 4, the embossing treatment is performed to join the thermally adhesive fabric layer 6 disposed on the upper surface side of the absorbing body 4 and the thermally adhesive fabric layer 6 disposed on the lower surface side of the absorbing body 4 to each other, so that the cellulose wadding of the absorbing article 4 can be prevented from being displaced or cracked while suppressing the diffusion of the bodily liquid in the widthwise directions of the absorbing body.

The embosses 7 are desirably formed in the stress directions of the absorbing bodies 4 thereby not to generate such a component force as to cause the cellulose wadding. In other words, the embosses are desirably formed along the direction, in which the underwear extends, or in which the absorbing body is liable to be cracked. In the example of the sanitary napkin 1 shown in Fig. 1, therefore, a plurality of lines of linear embosses 7 and 7 are added to the sanitary napkin 1 in a direction along the approximately longitudinal direction thereof. The linear embosses 7 and 7 are desired to have a spacing P of 1 to 30 mm, preferably 2 to 15 mm. In case, the spacing P of the linear embosses 7 and 7 is less than 1 mm, the absorbing bodies 4 are made too hard to satisfy the softness by the linear embosses 7 and 7. In case the spacing P of the linear embosses 7 and 7 is more than 30 mm, on the other hand, it is impossible to prevent the cellulose wadding from being displaced or cracked. On the other hand, the linear emboss 7 is desired to have a bottom groove width M of 0.05 to 5 mm, preferably 0.03 to 3 mm. In case the groove width M is more than 5 mm, the space portion of the groove is felt to deteriorate the wear feeling, and much pulverized pulp is interposed in the emboss portion between the upper and lower thermally adhesive fabric layers thereby to make it difficult to join the upper and lower thermally adhesive fabric layers to each other. In case the groove width is less than 0.05 mm, the groove width is so small that the thermally adhesive fabric layers (or the nonwoven fabric 6) are cut.

As schematically shown in the transverse section of Fig. 2, absorbing portions 4a sandwiched between the linear embosses 7 and 7 are confined in the bag-shaped spaces of the nonwoven fabrics 6 and 6, so that the cellulose wadding of the absorbing bodies 4 can be prevented from being displaced or cracked. Moreover, the nonwoven fabrics 6 and 6 are joined by the linear embosses 7 and 7 so that the pulp can also be prevented from being restored to increase its thickness.

At the same time, moreover, the bodily liquid to diffuse sideways penetrates into the absorbing bodies 4. When the bodily liquid reaches the joined portions (or the emboss portions 7 and 7), it is prevented, in case the nonwoven fabrics 6 and 6 are substantially directly joined, from diffusing any more sideways. In case the pulp of the absorbing bodies 4 is highly densified at the emboss portions, the bodily liquid diffuses along the highly densified emboss lines so that it is prevented from diffusing sideways.

Moreover, the individual linear embosses 7 and 7 act as hinge points so that the absorbing bodies 4 become easily deformable. As a result, their touches on the roots of the legs become weak so that the uncomfortable feeling is reduced to improve the wear feeling.

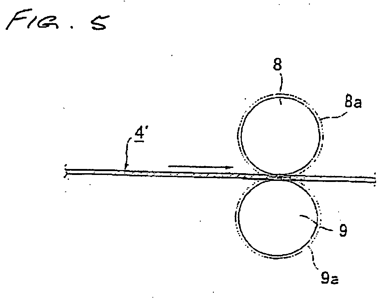

When the linear embosses 7 and 7 are to be formed on the absorbing body 4, an emboss roll 8 having multiple lines of ridges 8a circumferentially continuing on the roll surface and an anvil roll 9 having ridges 9a at positions corresponding to the ridges 8a are disposed to confront each other, as shown in Fig. 5. An absorbing strip 4' wrapped by the nonwoven fabric 6 is passed between the two rolls 8 and 9 so that the linear embosses 7 and 7 are continuously formed on the absorbing strip 4'.

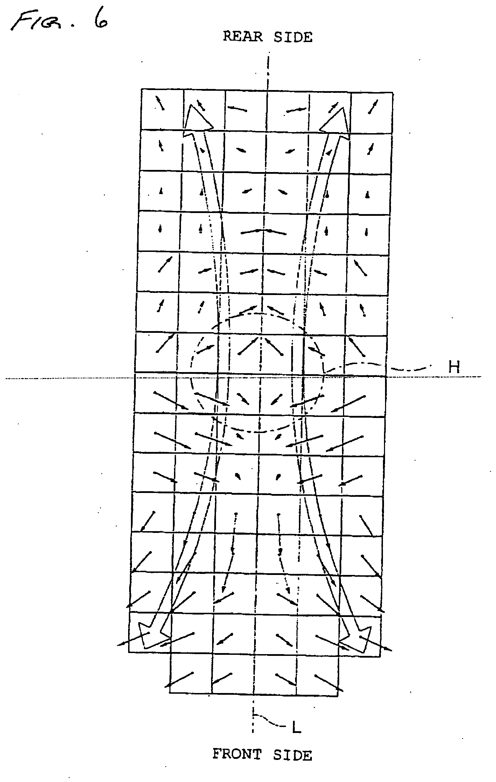

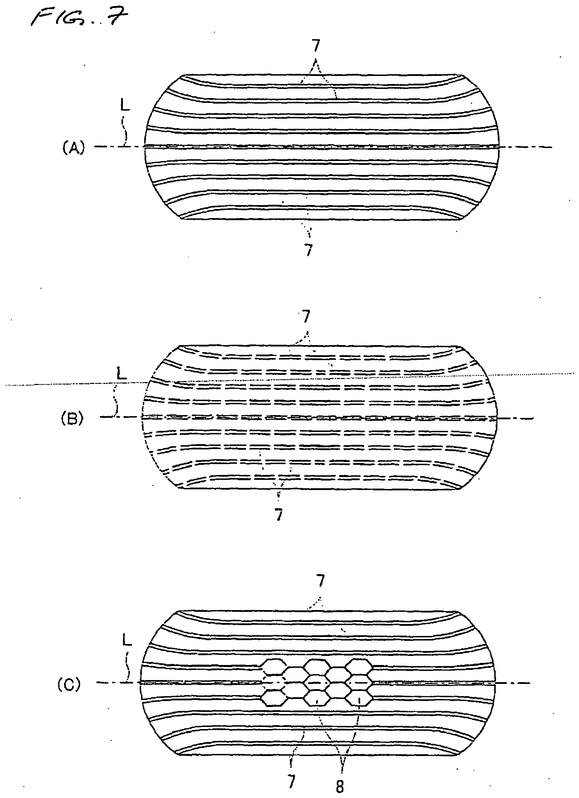

Incidentally, it is desired, as has been described hereinbefore, that the direction of the linear embosses 7 and 7 is identical to the extending direction of the shorts. The extension of the underwear, when worn by a dummy doll, was measured to provide the result, as shown in Fig. 6. The arrows in Fig. 6 indicate vectors having the "direction" and the "quantity". It is found from Fig. 6 that the vector lines are directed so individually outward at the front and rear end portions of a blood discharge portion H that they draw curves in directions to leave a longitudinal center line L gradually. As shown in Fig. 7, therefore, the linear embosses 7 and 7 are desired to have such patterns at the front and rear end portions of the absorbing body 4 that they draw patterns, in which they reach wholly or mostly the peripheral edge of the absorbing body 4 while drawing curves in the directions to leave the longitudinal center line L gradually outward. Fig. 7 (A) shows an example, in which the linear embosses 7 are continuous lines; Fig. 7(B) shows an example, in which the linear embosses are intermittent lines; and Fig. 7(C) shows an example, in which the honeycomb embosses 8 are applied to the vicinity of the blood discharge portion whereas the linear embosses 7 are applied to the remaining areas.

In order that the sanitary napkin 1 of the invention may make the softness to the skin and the prevention of the displacement and cracking of the cellulose wadding of the absorptive article compatible in a well-balanced manner, it is desired that the index for the softness to the skin, e.g., the WC value (or compression energy) by the KES compression tester is 0.8 gf · cm/cm² or higher, preferably 1.0 gf · cm/cm² or higher, or more preferably 1.2 gf · cm/cm² or higher, and that the index for the prevention of the displacement and cracking of the cellulose wadding of the absorptive article, e.g., the tensile strength at the wet time is 0.4 N or higher, or preferably 0.6 N or higher. As to the latter tensile strength at the wet time, the widthwise tensile strength of the absorbing body at the wet time is 0.4 N or higher, or preferably 0.6 N or higher, and the longitudinal tensile strength of the absorbing body at the wet time is 1.0 N or higher, or preferably 2.0 N or higher.

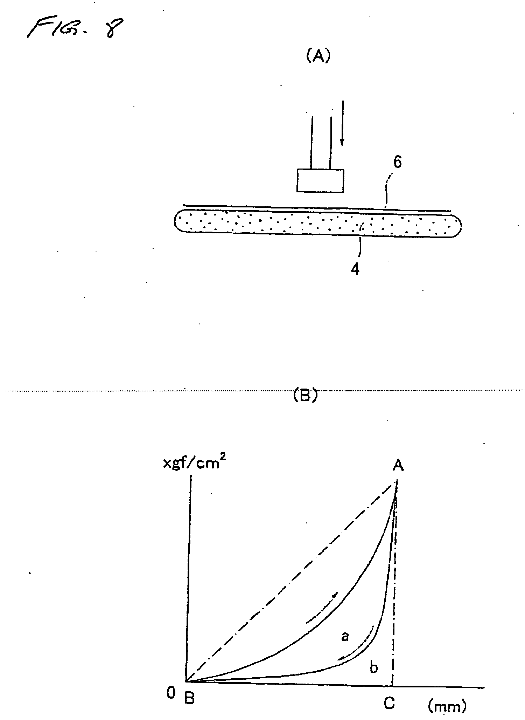

The KES compression tester [KATO TECH CO., LTD.] is a tester for simulating the feeling at the time when an object is touched by the finger of a person, and can measure the compression energy, the compression hardness, the compression recovery and so on. In the measurements, as shown in Fig. 8 (A), the sample is compressed under the conditions of a speed: 0.1 cm/sec, the compression area: 2 cm², the SENS: 2 (200g/10v by the power meter), the DEF sensitivity: 5, and the compression load: 50 gf/cm², and the LC (the compression hardness), the WC (the compression energy) and the RC (the compression recovery) are calculated from the correlation diagrams between the pressure and the deformation.

The LC (the compression hardness) is indicated as [the area of a + b] / [the area of a triangle ABC] in the correlation diagram of the pressure and the deformation, as shown in Fig. 8(B), and it is evaluated that the compression hardness is higher as the value comes closer to 1. The WC (or the compression energy) is indicated by [the area of a + b], and it is evaluated that the compression is easier for a higher value. The last RC (the compression recovery) is indicated by [the area of b]/[the area of a + b], and it is evaluated that the recovery is higher as the value comes closer to 100 %. Of these test items, the softness, as found by the sensory evaluation, employs the WC (the compression energy) as the index.

For the tests of the tensile strength, on the other hand, Tensilon RTC-1210A (ORIENTEC Co., LTD.) is used, and the maximum load value is adopted as the tensile strength by tensing a specimen having a length size of 40 mm and a width size of 20 mm at a tensing speed: 500 mm/min. in opposite directions.

### Examples

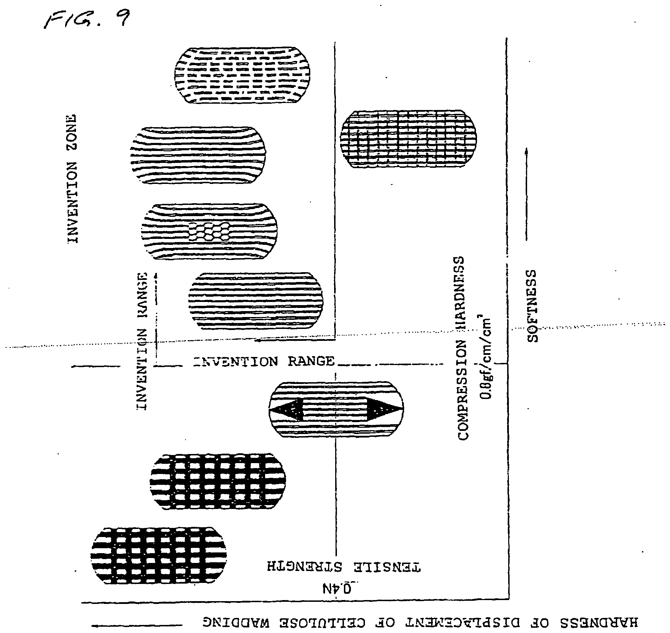

Specimens having various embosses are prepared and measured on softness by using the WC value (the compression energy) as the index, and the hardness of displacement of the cellulose wadding is measured by using the tensile strength test as the index. The results are shown in the graph of Fig. 9. In Fig. 9, the softness to the skin is higher in the rightward direction, and the displacement of the cellulose wadding is more difficult to occur in the upward direction. The four specimens, as exemplified in the embodiment, satisfy the conditions of the WC value (the compression energy) of 0.8 gf · cm/cm² or higher by the KES compression tester and the tensile strength at the wet time of 0.4 N or higher. It is found to satisfy the softness to the skin and the prevention of the displacement and the cracking of the cellulose wadding of the absorbing body in a well-balanced manner.

### (Other Modes of Embodiment)

(1) In the thin sanitary napkin 1 shown in Fig. 1, it is arbitrary to dispose the hydrophilic fiber layer as the so-called "second sheet" so partially as to limit the area such as a blood discharge portion, or to dispose the same all over the absorbing body 4.

### Brief Description of the Drawings

[Fig. 1] A partially broken perspective view of a thin sanitary napkin 1 according to the invention.
[Fig. 2] A partially enlarged transverse section of the same.
[Fig. 3] Both (A) and (B) diagrams showing modes of arrangement of a nonwoven fabric 6.
[Fig. 4] A transverse section showing another mode of the nonwoven fabric 6.
[Fig. 5] A diagram for applying linear embosses 7.
[Fig. 6] A vector diagram showing the extending state of an underwear when worn.
[Fig. 7] All (A) to (C) top plan views showing examples of other linear embosses 7 and 7.
[Fig. 8] (A) a testing procedure by a KES compression tester, and (B) a correlation diagram showing the KES compression test results between the pressure and the deformation.
[Fig. 9] A correlation diagram of individual napkins in case the abscissa indicates softness whereas the ordinate indicates the hardness of displacement of cellulose wadding.

### Description of Reference Numerals and Signs

1 ... Thin Sanitary Napkin, 2 ... Liquid Impermeable Rear Surface Sheet, 3 ... Liquid Permeable Front Surface Sheet, 4 ... Absorbing Body, 6 ... Thermally Adhesive Fabric Layer (Nonwoven Fabric), and 7 ... Linear Embosses.

## Claims

1. An absorptive article comprising press-thinned absorbing bodies interposed between a liquid permeable front surface sheet and a liquid impermeable rear surface sheet, said press-thinned absorbing bodies comprising pulp and a highly water-absorptive resin,
**characterized in that**
thermally adhesive fabric layers are disposed individually on the upper and lower surface sides of said press-thinned absorbing bodies, and a plurality of lines of linear embosses are provided in a direction along the approximately longitudinal direction of said absorptive article, which embosses join the thermally adhesive fabric layer disposed on the upper surface side of the press-thinned absorbing bodies and the thermally adhesive fabric layer disposed on the lower surface side of the press-thinned absorbing bodies to each other and said plural lines of linear embosses reach wholly or mostly the peripheral edge of the press-thinned absorbing body at the front and rear end portions of the press-thinned absorbing body and form curves in directions gradually diverging from a longitudinal center line of the absorptive article.

2. An absorptive article as set forth in claim 1, wherein said plural lines of linear embosses have a spacing of 1 to 30 mm, and wherein the embosses have a bottom groove width of 0.05 to 5 mm.

3. An absorptive article as set forth in any of claims 1 to 2, wherein a nonwoven fabric is disposed as said thermally adhesive fabric layers, and wherein said press-thinned absorbing body is peripherally wrapped by a single sheet.

4. An absorptive article as set forth in any of claims 1 to 3, wherein the pulp is highly densified at portions of the thermally adhesive fabric layers joined to each other by the embosses.

## Patentansprüche

1. Absorptionsfähiger Gegenstand, der zwischen eine flüssigkeitsdurchlässige vorderseitige Oberflächenlage und eine flüssigkeitsundurchlässige rückseitige Oberflächenlage eingefügte dünngepresste Absorptionskörper umfasst, wobei die dünngepressten Absorptionskörper Zellstoff und ein stark wasserabsorbierendes Harz umfassen,
**dadurch gekennzeichnet, dass** wärmeklebende Gewebeschichten individuell auf der oberen und der unteren Oberflächenseite der dünngepressten Absorptionskörper angeordnet sind und eine Mehrzahl von Linien linearer Prägungsbereiche in einer Richtung längs der näherungsweisen Längsrichtung des absorptionsfähigen Gegenstands angeordnet sind, wobei diese Prägebereiche die auf der oberen Oberflächenseite der dünngepressten Absorptionskörper angeordnete wärmeklebende Gewebeschicht und die auf der unteren Oberflächenseite der dünngepressten Absorptionskörper angeordnete wärmeklebende Gewebeschicht miteinander verbinden und die mehreren Linien linearer Prägebereiche den Umfangsrand des dünngepressten Absorptionskörpers an den vorderen und rückwärtigen Endbereichen des dünngepressten Absorptionskörpers vollständig oder größtenteils erreichen und Kurven in Richtungen, die von einer longitudinalen Mittellinie des absorptionsfähigen Gegenstands aus allmählich auseinanderlaufen, bilden.

2. Absorptionsfähiger Gegenstand gemäß Anspruch 1, wobei die mehreren Linien linearer Prägebereiche einen Abstand von 1 bis 30 mm aufweisen und wobei die Prägebereiche eine untere Rillenbreite von 0,05 bis 5 mm aufweisen.

3. Absorptionsfähiger Gegenstand gemäß einem der Ansprüche 1 bis 2, wobei ein Vlies als die wärmeklebenden Gewebeschichten angeordnet ist, und wobei der dünngepresste Absorptionskörper am Umfang durch eine einzige Lage eingewickelt ist.

4. Absorptionsfähiger Gegenstand gemäß einem der Ansprüche 1 bis 3, wobei der Zellstoff in Bereichen der wärmeklebenden Gewebeschichten, die durch die Prägebereiche miteinander verbunden sind, stark verdichtet ist.

## Revendications

1. Article absorbant comprenant des corps absorbants amincis à la presse interposés entre une feuille de surface avant perméable aux liquides et une feuille de surface arrière imperméable aux liquides, lesdits corps absorbants amincis à la presse comprenant une pâte et une résine à capacité d'absorption d'eau élevée,
**caractérisé en ce que**
des couches d'étoffe thermiquement adhésives sont disposées individuellement sur les côtés de surfaces supérieure et inférieure desdits corps absorbants amincis à la presse, et une pluralité de lignes de bossages linéaires sont fournies dans une direction le long de la direction approximativement longitudinale dudit article absorbant, lesquels bossages joignent la couche d'étoffe thermiquement adhésive disposée sur le côté de surface supérieure des corps absorbants amincis à la presse et la couche d'étoffe thermiquement adhésive disposée sur le côté de surface inférieure des corps absorbants amincis à la presse l'une à l'autre et lesdites plusieurs lignes de bossages linéaires atteignent intégralement ou majoritairement le bord périphérique du corps absorbant aminci à la presse au niveau des portions d'extrémité avant et arrière du corps absorbant aminci à la presse et forment des courbes dans des directions divergeant progressivement depuis une ligne de centre longitudinale de l'article absorbant.

2. Article absorbant selon la revendication 1, dans lequel lesdites plusieurs lignes de bossages linéaires ont un espacement de 1 à 30 mm, et dans lequel les bossages ont une largeur de rainure de dessous de 0,05 à 5 mm.

3. Article absorbant selon l'une quelconque des revendications 1 à 2, dans lequel une étoffe non tissée est disposée en tant que lesdites couches d'étoffe thermiquement adhésives, et dans lequel ledit corps absorbant aminci à la presse est périphériquement enveloppé par une seule feuille.

4. Article absorbant selon l'une quelconque des revendications 1 à 3, dans lequel la pâte est hautement densifiée en des portions des couches d'étoffe thermiquement adhésives jointes les unes aux autres par les bossages.
